(19) Europäisches Patentamt
European Patent Office
Office européen des brevets

(11) Veröffentlichungsnummer : **0 135 011 B1**

(12) # EUROPÄISCHE PATENTSCHRIFT

(45) Veröffentlichungstag der Patentschrift : **02.11.89**

(51) Int. Cl.$^4$ : **A 61 K 7/08**

(21) Anmeldenummer : **84108278.7**

(22) Anmeldetag : **13.07.84**

(54) **Mittel zum Waschen und Spülen von Haaren.**

(30) Priorität : **21.07.83 DE 3326230**

(43) Veröffentlichungstag der Anmeldung : **27.03.85 Patentblatt 85/13**

(45) Bekanntmachung des Hinweises auf die Patenterteilung : **02.11.89 Patentblatt 89/44**

(84) Benannte Vertragsstaaten : **AT BE CH DE FR GB IT LI NL SE**

(56) Entgegenhaltungen :
**DE-A- 2 044 601**
**DE-A- 2 452 031**
**DE-A- 2 452 032**
**GB-A- 2 028 133**
**US-A- 4 381 259**

(73) Patentinhaber : **Henkel Kommanditgesellschaft auf Aktien**
**Postfach 1100 Henkelstrasse 67**
**D-4000 Düsseldorf-Holthausen (DE)**

(72) Erfinder : **Giede, Karl**
**Schlehenweg 12**
**D-4010 Hilden (DE)**
Erfinder : **Höffkes, Horst, Dr.**
**Carlo-Schmid-Strasse 113**
**D-4000 Düsseldorf 13 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 135 011 B1**

## Beschreibung

Gegenstand der Erfindung sind Mittel zum Waschen oder Spülen von Haaren, also z. B. Shampoos und abspülbare Haarnachbehandlungsmittel, mit einem, die Fülle und Frisierbarkeit der Haare verbessernden Zusatz.

Das Haupthaar weist nach dem Waschen oft einen kosmetisch unbefriedigenden Zustand auf. Es fühlt sich stumpf an, ist im nassen Zustand nur schwer zu kämmen und neigt im trockenen Zustand zur statischen Aufladung, wodurch das Kämmen ebenfalls erschwert und der Sitz des gekämmten Haares gestört wird.

Es ist daher bekannt, nach dem Waschen oder Schamponieren des Haares konditionierende Präparate, meist mit kationischen grenzflächenaktiven Stoffen, einwirken zu lassen oder den Haarwaschmitteln konditionierende Stoffe zuzusetzen, um gleichzeitig mit der Haarwäsche einen gewissen konditionierenden Effekt zu erzielen. Solche Substanzen sind z. B. wasserlösliche Proteine, Proteinabbauprodukte oder polykationische Polymere, z. B. kationische Cellulosederivate.

Während mit bekannten Hilfsmittelneine befriedigende Verbesserung der Naßkämmbarkeit und, z. B. mit kationischen Tensiden, auch eine Verringerung der antistatischen Aufladung erzielt wird, ist die praktisch immer gleichzeitig erzielte zu starke Glättung des trockenen Haares sehr nachteilig denn sie bewirkt, daß das Haar wenig Fülle und die Frisur keinen Halt aufweist. Die Glätte des Haares ist um so ausgeprägter, je niedriger der Kämmwiderstand des trockenen Haares ist.

Es bestand daher die Aufgabe, Haarwaschmittel und Haarspülmittel aufzufinden, welche die Glätte des trockenen Haares merklich verringern, ohne eine Klebrigkeit des Haares zu verursachen und ohne die Naßkämmbarkeit zu sehr zu verringern.

Es ist aus DE-OS-23 10 283 bekannt, Haarshampoos Polymerisate von Acrylsäure, Methacrylsäure, Crotonsäure, Ithaconsäure und Maleinsäure sowie deren Copolymerisate mit einem monoäthylenisch ungesättigten Monomeren, insbesondere Ethylen, Vinylbenzol, Vinylacetat, Vinylmethyläther und Acrylamid zuzusetzen. Diese Produkte erniedrigen aber den Kämmwiderstand des trockenen Haares, d. h. sie lösen nicht die Aufgabe der vorliegenden Erfindung.

In DE-A-24 52 031 ist die Verwendung von Polyaldehydocarbonsäuren als Filmbildner in Haarsprays und Festigern beschrieben. Aus DE-A-20 44 610 war die Verwendung von Polyaldehydocarbonsäuren als Ersatz für Phosphat-Buildersubstanzen in Textilwaschmitteln bekannt.

Es wurde gefunden, daß die Verwendung einer wäßrigen Zubereitung mit einem Gehalt an Polyaldehydocarbonsäuren mit Monomereinheiten der allgemeinen Formeln I und II,

$$-CH_2-\underset{\displaystyle CHO}{\underset{|}{CH}}- \quad (I) \qquad\qquad -CH_2-\underset{\displaystyle COOH}{\underset{|}{CH}}- \quad (II)$$

die ein mittleres Molekulargewicht von 600 bis 10 000 haben und 5 bis 9, bevorzugt von 7 bis 9, Carboxylgruppen und 1 bis 5, bevorzugt 1 bis 3, Aldehydgruppen pro 10 Monomereinheiten aufweisen oder deren wasserlöslichen Salzen zum Waschen oder Spülen von Haaren die Glätte des trockenen Haares verringert und die Naßkämmbarkeit des Haares nur unwesentlich reduziert.

Weiterhin wurde gefunden, daß gleichzeitig die Naßkämmbarkeit des Haares verbessert und die Glätte des trockenen Haares verringert werden kann, wenn in Mitteln zum Waschen oder Spülen der Haare 0,1-10 Gew.-% an Polyaldehydocarbonsauren mit Monomereinheiten der Formeln I und II oder deren wasserlöslichen Salzen und 0,1-10 Gew.—% einer anionischen haaravivierenden Komponente, ausgewählt aus Verbindungen der allgemeinen Formeln $RO(CH_2—CH_2—O)_nPO_3A_2$, $[RO(CH_2—CH_2—O)_n]_2PO_2A$, $RPO_3A_2$, $R—CH(OH)PO_3A_2$, $R—CX(PO_3A_2)_2$, in denen R eine lineare Alkylgruppe mit 8-18 C-Atomen, X Wasserstoff, eine OH- oder eine $NH_2$-Gruppe, A ein Alkali-, Ammonium-, Mono-, Di- oder Triethanolammonium-Kation und n = 0 oder eine ganze Zahl von 1-12 ist, enthalten sind.

Die in den Polyaldehydocarbonsäuren enthaltenen Monomereinheiten der allgemeinen Formel I und II können in beliebiger Reihenfolge angeordnet sein. Das mittlere Molekulargewicht der Polyaldehydocarbonsäuren soll zwischen 600 und 10 000 liegen, dies entspricht einem mittleren Polymerisationsgrad von ca. 10-140. Das Verhältnis von Aldehydgruppen zu Carboxylgruppen soll 5 : 5 bis 1 : 9 betragen, d. h. es sollen etwa 5-9 Carboxylgruppen und 1-5 Aldehydgruppen pro 10 Monomereinheiten vorhanden sein.

Bevorzugt geeignete Polyaldehydocarbonsäuren enthalten 7-9 Carboxylgruppen und 1-3 Aldehydgruppen pro 10 Monomereinheiten. Solche Polyaldehydocarbonsäuren sind bekannte Handelsprodukte. Sie werden z. B. durch eine oxidative Homopolymerisation von Acrolein oder auch durch oxidative Copolymerisation von Acrolein und Acrylsäure hergestellt. Handelsübliche Polyaldehydocarbonsäuren sind z. B. die Produkte der Firma DEGUSSA

— POC-HS-0010, eine 50 %ige wäßrige Lösung einer Polyaldehydo-Carbonsäure, mittleres Molekulargewicht 700, Säurezahl, Säureäquivalentgewicht 102,

— POC-HS-2020, eine 50 %ige wäßrige Lösung einer Polyaldehydocarbonsäure, mittleres Molekulargewicht 1400, Säurezahl 585, Säureäquivalentgewicht 96,

— POC-HS-5060, eine 40 %ige wäßrige Lösung einer Polyaldehydocarbonsäure, mittleres Molekulargewicht 4000, Säurezahl 645, Säureäquivalentgewicht 87,

— POC-HS-65120, eine 35 %ige wäßrige Lösung einer Polyaldehydocarbonsäure, mittleres Molekulargewicht 8500 Säurezahl 695, Säureäquivalentgewicht 81.

Die als weitere Zusätze zu den erfindungsgemäßen Mitteln geeigneten anionischen, oberflächenaktiven, haaravivierenden Komponenten der allgemeinen Formeln $RO(CH_2—CH_2—O)_nPO_3A_2$ und $[RO(CH_2—CH_2O)_n]_2—PO_2A$ sind Salze von Phosphorsäuremono- und -diestern von linearen $C_8$-$C_{18}$-Alkanolen und von Anlagerungsprodukten von 1-12 Mol Ethylenoxid an lineare $C_8$-$C_{18}$-Alkanole. Solche Produkte werden durch Umsetzung der Alkanole oder der Ethylenoxidanlagerungsprodukte an die Alkanole mit Phosphorpentoxid und anschließende Neutralisation mit wäßrigen Alkalihydroxiden, Ammoniak oder Alkanolaminen hergestellt.

Die als weitere Zusätze geeigneten, anionischen, oberflächenaktiven, $C_8$-$C_{18}$-Alkan-1-phosphonsäuren und deren Salze sind bekannt oder nach literaturbekannten Verfahren herstellbar. Sie können z. B. gemäß US-PS-2.957.931 durch radikalische Anlagerung von Estern der phosphorigen Säure an Olefine und nachfolgende Verseifung der Alkan-1-phosphonsäureester hergestellt werden.

1-Hydroxyalkan-1-phosphonsäuren können z. B. nach dem von W. Fossek im Monatshefte für Chemie, Bd. 7 (1886), Seite 20-39 angegebenen Verfahren durch Umsetzung von Aldehyden mit $PCl_3$ dargestellt werden.

Die Alkan-1.1-diphosphonsäuren können z. B. durch Akylierung von Methandiphosphonsäure nach dem Verfahren hergestellt werden, welches G.M. Kosolapoff in J. Am. Chem. Soc. 75 (1975), Seite 1500-1501 für die Pentan-1.1-diphosphonsäure angegeben hat.

1-Hydroxyalkan-1.1-diphosphonsäuren sind nach den von B. Blaser et al. in der Zeitschrift für anorganische und allgemeine Chemie, Bd. 381 (1971) Seite 247-259 beschriebenen Verfahren aus Carbonsäuren, Wasser und $PCl_3$ zugänglich.

1-Aminoalkan-1.1-diphosphonsäuren können nach einem von W. Plöger et al. in der Zeitschrift für anorganische und allgemeine Chemie, Bd. 389 (1972), Seite 119-128 beschriebenen Verfahren hergestellt werden.

Bevorzugt geeignete, anionische oberflächenaktive haaravivierende Komponenten sind die $C_8$-$C_{18}$-Alkan-1-phosphonsäuren, ganz besonders die im Handel erhältliche Octan-1-phosphonsäure und deren Natriumsalz.

Erfindungsgemäße Mittel zum Waschen der Haare, die zur Anwendung als Haarshampoo geeignet sind, enthalten bevorzugt 1-10 Gew.-% der Polyaldehydocarbonsäuren und 5-50 Gew.-% anionischer Tenside, die ausgewählt sind aus Verbindungen der allgemeinen Formeln $R$-$So_3A$ und $RO(CH_2—CH_2O)_n—SO_3A$, in denen R eine lineare Alkylgruppe mit 8-18 C-Atomen, A ein Alkali-, Amonium-, Mono-, Di- oder Triethanolammonium-Kation und $n = 0$ ist oder eine ganze Zahl von 1-12 darstellt.

Geeignete anionische Tenside dieser Gruppen sind z. B. die Natriumsalze von Alkansulfonaten mit 12-18 C-Atomen, die Ammonium-oder Alkanolammoniumsalze von Alkylsulfaten auf Basis von linearen oder wenig verzweigten Fettalkoholen mit 12-16 C-Atomen und die Natrium-, Ammonium-, Mono-, Di- oder Triethanolammoniumsalze von Alkyl(poly)glycolethersulfaten mit 12-16 C-Atomen in der Alkylgruppe und 1-6 Glycolethergruppen.

Besonders vorteilhaft ist die Anwendung einer Mischung aus 5-20 Gew.-% eines anionischen Tensides vom Typ des $C_{12}$-$C_{16}$-Fettalkohol-(poly)-glycolethersulfats mit 1-6 Glycolethergruppen und 1-10 Gew.-% eines ampholytischen Tensides, eines Betaintensides oder eines Aminoxid-Tensides, bezogen auf das gesamte Shampoo.

Als ampholytische Tenside können z. B. N-$C_8C_{18}$-Alkyl-β-aminopropionsäuren, N-$C_8$-$C_{18}$-Alkyl-β-iminodipropionsäuren oder N-Hydroxyethyl-N-Kokosalkylamidopropyl-glycin eingesetzt werden. Geeignete Betaintenside sind z. B. N-Kokosalkyl-dimethyl-glycin und N-Kokosalkylamidopropyl-dimethyl-glycin. Als Aminoxid-Tenside sind z. B. N-Kokosalkylamidopropyl-dimethylamin-oxid oder N-Kokosalkyl-di(2-hydroxy)ethylamin-oxid geeignet.

Daneben können die erfindungsgemäßen Haarwaschmittel übliche Zusatz- und Stellmittel wie z. B. Verdickungsmittel mit Schaumstabilisatoren von der Art der Fettsäurealkanolamide, Trübungsmittel, z. B. von der Art des Ethylenglycoldistearats, PH-Wert-Stabilisatoren bzw. Puffersysteme wie Alkali- oder Ammonium-Phosphate oder Citrate, Konservierungsmittel wie Formaldehyd oder p-Hydroxybenzoesäureester, Farbstoffe, Duftstoffe sowie bekannte haarkosmetische Wirkstoffe wie Antischuppenmittel oder Sebostatika enthalten.

Erfindungsgemäße Mittel zum Spülen der Haare, die zur Anwendung als konditionierende Haarnachbehandlungsmittel wie z. B. Haarspülungen, Haarkurpräparate geeignet sind, enthalten bevorzugt 0,1-10 Gew.-% der Polyaldehydocarbonsäuren und 1-10 Gew.% einer anionischen, haaravivierenden Komponente, ausgewählt aus Verbindungen der allgemeinen Formeln $RO(CH_2—CH_2—O)_nPO_3A_2$, $[RO(CH_2—CH_2—O)_n]_2PO_2A$, $R—PO_3A_2$, $R—CH(OH)PO_3A_2$, $RCX(PO_3A_2)_2$ in denen R eine lineare Ammonium-, Mono-, Di- oder Triethanolammonium-Kation und $n = 0$ oder eine ganze Zahl von 1-12 ist.

Bevorzugt werden wasserlösliche Salze von Phosphorsäure-mono- und diestern linearer $C_{12}$-$C_{18}$-Alkylgruppe mit 8-18 C-Atomen, X = Wasserstoff, eine OH- oder eine $NH_2$-Gruppe, A ein Alkali-,

Fettalkohole und von $C_8$-$C_{18}$-Alkan-1-phosphonsäuren eingesetzt. Ein besonders geeignetes Alkan-1-phosphonat ist das Natriumsalz der Octan-1-phosphonsäure. Daneben können die erfindungsgemäßen Haarspülmittel übliche Zusatz- und Stellmittel wie z. B. Verdickungsmittel von der Art der wasserlöslichen anionischen oder nichtionogenen Cellulose-Stärke- oder Guarderivate, Alginate, wasserlösliche verdickende synthetische Polymerisate wie z. B. Polyvinylalkohol, Polyvinyl-pyrrolidon, Polyacrylate, Polyacrylamide usw. enthalten. Besonders geeignet ist der Einsatz von 1-5 Gew.-% eines wasserlöslichen, nichtionogenen Cellulosederivats, insbesondere von Hydroxyethylcellulose zur Verdickung der erfindungsgemäßen Haarspülmittel. Sie können zur Erlangung einer cremigen Struktur auch wäßrige Dispersionen oder Emulsionen von Fettalkoholen und/oder kosmetischen Ölkomponenten sowie die zur Herstellung solcher Dispersionen erforderlichen Emulgatoren enthalten. Daneben könne die Haarspülmittel Konservierungsstoffe, Duftstoffe, Farbstoffe und bekannte haarkosmetische Wirkstoffe wie Kräuterextrakte, Vitamine, Balsame, Antischuppenwirkstoffe oder Sebostatika enthalten.

Die nachfolgenden Beispiele sollen den Gegenstand der Erfindung näher erläutern.

## Beispiele

1. Beeinflussung der Naß- und Trockenkämmbarkeit

Es wurde ein Standard-Shampoo ohne und mit einem Zusatz von 1,0 Gew.-% verschiedener Polyaldehydocarbonsäuren sowie ein reines Alkylethersulfattensid vergleichend in bezug auf die Naßkämmbarkeit und die Trockenkämmbarkeit untersucht.

| Prüfshampoo-Rezepturen | A | B | C | D | E |
|---|---|---|---|---|---|
| Fettalkohol $C_{12/14}$ + 2 EO-sulfat-Na-Salz 28 Gew.-%ig (Texapon® N 25) | 50 | 50 | 50 | 50 | 50 |
| N-Kokosalkylamidopropyldimethylglycin 30 Gew.-%ig (Dehyton® K) | - | 5 | 5 | 5 | 5 |
| Wollfettalkoholoxethylat | - | 1 | 1 | 1 | 1 |
| Ethylenglycol tearat | - | 2 | 2 | 2 | 2 |
| Parfümöl | - | 0,5 | 0,5 | 0,5 | 0,5 |
| Ammoniak-Lösung bis pH = 5 | - | - | 0,5 | 0,5 | 0,5 |
| POC-HS-2020 (50 Gew.-%ig) | - | - | 2,0 | - | - |
| POC-HS-5060 (40 Gew.-%ig) | - | - | - | 3,3 | - |
| POC-HS-65120 (35 Gew.-%ig | - | - | - | - | 3,3 |
| Wasser | 50 | 41,5 | 39 | 37,7 | 37,7 |
| % NK | 100 | 129 | 125 | 134 | 165 |
| % TK | 100 | 106 | 155 | 127 | 138 |

1.1 Bestimmung der Naßkämmbarkeit (NK) und der Trockenkämmbarkeit (TK)

0,7 Gramm schwere, ungeschädigte, dunkelbraune, europäische Haarsträhnen wurden 5 Minuten lang bei 30 °C mit Lösung A gereinigt, anschließend mit Wasser von 30 °C tensidfrei gespült, dann zweimal 15 Minuten lang im Ultraschallbad mit Leitungswasser von 22 °C behandelt. Die so vorkonditionierten Haarsträhnen wurden mit den Prüfshampooformulierungen A-E 5 Minuten lang bei 30 °C behandelt, anschließend mit lauwarmem Wasser (30 °C) gründlich ausgespült, naß ausgerichtet, überschüssiges Wasser abgestreift und bei 40 °C in trockenem Luftstrom 16 Std. bei 25 °C getrocknet.

a) Trockenkämmbarkeit (TK)

Zur Prüfung der Trockenkämmbarkeit wurden die Haarsträhnen 16 Stunden bei 25 °C und 60 % relativer Luftfeuchte konditioniert.

b) Naßkämmbarkeit (NK)

Zur Prüfung der Naßkämmbarkeit wurden die Haarsträhnen mit 50 Gew.-% des Haargewichtes an Wasser befeuchtet.

Dann erfolgte die Messung des Kämmwiderstandes, d. h. der Kraft, die erforderlich war, um einen Kamm durch eine Haarsträhne zu ziehen. Zur Verringerung der Meßfehlerbreite erfolgte die Bestimmung für jede Formulierung an 3 verschiedenen Haarsträhnen über jeweils 5 Kämmungen. Von den erhaltenen Arbeitsintegralen wurden die Mittelwerte gebildet. Die Messung des Kämmwiderstandes erfolgte mit einer Zug-Prüfmaschine des Typs 1402 der Firma Zwick (Einsingen über Ulm/Donau) Das mittlere Arbeitsintegral wurde auf das der mit Lösung A (Blindwert) behandelten Haare bezogen. Die Kämmwiderstands-Erniedrigung bzw. -erhöhung ergibt sich damit aus der Beziehung

$$\text{NK bzw. TK} = \frac{\text{ø Arbeitsintegral Prüflösung}}{\text{ø Arbeitintegral Lösung A}} \times 100\ [\%]$$

Werte unter 100 % bedeuten demnach eine Erniedrigung, Werte über 100 % eine Erhöhung des mittleren Kämmwiderstandes.

1.2 Ergebnis :

Aus den Meßdaten ist ersichtlich, daß der Zusatz der Polyaldehydocarbonsäuren POC-HS 2020 (mittleres Molekulargewicht 1400) und POC-HS-5060 (mittleres Molekulargewicht 4000) die Naßkämmbarkeit des Prüfshampoos B nur wenig verändern, den Trockenkämmwiderstand hingegen erheblich erhöhen.

Der Zusatz der Polyaldehydocarbonsäure POC-HS 65120 (mittleres Molekulargewicht 8500) erhöht sowohl den Naß- als auch den Trockenkämmwiderstand. Durch den Zusatz der Polyaldehydocarbonsäuren bzw. deren Salzen wird die Glätte des trockenen Haares verringert.

2. Anwendungsbeispiele

2.1 Haarshampoo-Rezepturen

| | 2.1.1 | 2.1.2 | 2.1.3 | 2.1.4 | 2.1.5 | 2.1.6 |
|---|---|---|---|---|---|---|
| - Fettalkohol $C_{12/14}$+ 2EO-sulfat, Na-Salz, 28 %ig (Texapon® N25) | 50 | 35 | 50 | 50 | 60 | 40 |
| - Octan-1-phosphonsäure | - | - | - | - | - | 1,0 |
| - N-Kokosalkylamidopropyl--dimethylglycin, 30%ig (Dehyton®K) | 10 | - | - | - | - | 10 |
| - Cocoamphoglycinat 30%ig (Dehyton®G) | - | 15 | - | - | - | - |
| - N-Kokosalkylamidopropyl-dimethyl-aminoxid 35%ig (Aminoxid WS 35) | - | - | 10 | - | - | - |
| - Kokosfettsäurediethanolamid | 2 | 2 | 2 | 2 | 2 | 2 |
| - Abietinsäure-Eiweiß-Kondensationsprodukt (Lamepon®PATR) | - | - | - | 5 | - | - |
| - Ethylenglycoldistearat | 2 | 2 | 2 | 2 | 2 | 2 |
| - Polyaldehydocarbonsäure, 40%ig (POC-HS-5060) | 5 | 5 | 5 | 5 | 5 | 6 |
| - Triethanolamin bis pH=6 | ca.2 | ca.2 | ca.2 | ca.2 | ca.2 | ca.3 |
| - Wasser, Parfüm, Farbstoffe | ad100 | ad100 | ad100 | ad100 | ad100 | ad100 |

2.2 Haarspülung

| | |
|---|---|
| Polyaldehydocarbonsäure, 40 %ig (POC-HS-5060) | 2,0 Gew.-% |
| Octylphosphonsäure | 2,5 Gew.-% |
| Hydroxyethylcellulose | 1,2 Gew.-% |
| Natronlauge, 10 %ig | bis pH = 4,5 |
| Wasser, Parfüm, Farbstoff | ad 100 % |

2.3 Haarkur-Gel

| | |
|---|---|
| Polyaldehydocarbonsäure, 50 %ig (POC-HS-2020) | 5,0 Gew.-% |
| Kokosalkylphosphat, Na-Salz, 15 %ig (Forlanit® F 452) | 3,0 Gew.-% |
| Hydroxyethylcellulose | 2,0 Gew.-% |
| Triethanolamin | bis pH = 4,5 |
| Wasser, Parfüm, Farbstoff | ad 100 % |

**Patentansprüche**

1. Verwendung einer wäßrigen Zubereitung mit einem Gehalt an Polyaldehydocarbonsäuren mit Monomereinheiten der allgemeinen Formeln I und II,

$-CH_2-CH-$ with CHO (I) $-CH_2-CH-$ with COOH (II)

die ein mittleres Molekulargewicht von 600 bis 10 000 haben und 5 bis 9, bevorzugt von 7 bis 9, Carboxylgruppen und 1 bis 5, bevorzugt 1 bis 3, Aldehydgruppen pro 10 Monomereinheiten aufweisen oder deren wasserlöslichen Salzen zum Waschen oder Spülen von Haaren.

2. Mittel zum Waschen oder Spülen der Haare in Form einer wäßrigen Zubereitung mit einem Gehalt von 0,1 bis 10 Gew.-% an Polyaldehydocarbonsäuren mit Monomereinheiten der allgemeinen Formeln I und II,

$-CH_2-CH-$ with CHO (I) $-CH_2-CH-$ with COOH (II)

die ein mittleres Molekulargewicht von 600 bis 10 000 haben und 5 bis 9, bevorzugt 7 bis 9, Carboxylgruppen und 1 bis 5, bevorzugt 1 bis 3, Aldehydgruppen pro 10 Monomereinheiten aufweisen, oder deren wasserlöslichen Salzen, dadurch gekennzeichnet, daß zusätzlich 0,1 bis 10 Gew.-% einer anionischen haaravivierenden Komponente, ausgewählt aus Verbindungen der allgemeinen Formeln $RO—(CH_2—CH_2O)_n—PO_3A_2$, $[RO(CH_2CH_2O)_n]_2PO_2A$, $R—PO_3A_2$, $R—CH(OH)PO_3A_2$, $RCX(PO_3A_2)_2$, in denen R eine lineare Alkylgruppe mit 8 bis 18 C-Atomen, X Wasserstoff, eine OH- oder eine $-NH_2$-Gruppe, A ein Alkali-, Ammonium-, Mono-, Di-, oder Triethanolammonium-Kation und n = 0 oder eine ganze Zahl von 1 bis 12 ist, enthalten sind.

3. Mittel nach Anspruch 2 zur Anwendung als Haarshampoo, dadurch gekennzeichnet, daß 1 bis 10 Gew.-% der Polyaldehydocarbonsäure und 5 bis 50 Gew.-% anionischer Tenside, ausgewählt aus Verbindungen der allgemeinen Formeln $R-SO_3-A$, und/oder $RO(CH_2—CH_2—O)_n—SO_3A$, in denen R eine lineare Alkylgruppe mit 8 bis 18 C-Atomen, A ein Alkali-, Ammonium- oder Alkanolammoniumkation und n = 0 oder eine ganze Zahl von 1 bis 12 ist, enthalten sind.

4. Mittel nach Anspruch 3, dadurch gekennzeichnet, daß als anionisches Tensid ein $C_{12}$-$C_{16}$-Fettalkohol(poly)-glykolethersulfat-Natriumsalz mit 1 bis 6 Glykolethergruppen in einer Menge von 5 bis 20 Gew.-% und zusätzlich ein ampholytisches Tensid, ein Betaintensid oder ein Aminoxid-Tensid in einer Menge von 1 bis 10 Gew.-% enthalten ist.

5. Mittel nach Anspruch 2 zur Anwendung als Haarnachspülmittel, dadurch gekennzeichnet, daß 1 bis 10 Gew.-% einer anionischen, haaravivierenden Komponente ausgewählt aus den wasserlöslichen Salzen von Phosphorsäure-mono- und -diestern linearer $C_{12}$-$C_{18}$-Fettalkohole und/oder von $C_8$-$C_{18}$-Alkan-1-Phosphonsäuren enthalten sind.

## Claims

1. The use of an aqueous preparation with a content of polyaldehydocarboxylic acids which contain monomer units corresponding to general formulae I and II

$$-CH_2-\underset{CHO}{\underset{|}{CH}}- \quad (I) \qquad -CH_2-\underset{COOH}{\underset{|}{CH}}- \quad (II)$$

and which have have an average molecular weight of 600 to 10,000 and contain from 5 to 9 and preferably from 7 to 9 carboxyl groups and from 1 to 5 and preferably from 1 to 3 aldehyde groups per 10 monomer units or water-soluble salts thereof for washing or rinsing hair.

2. Hair washing or rinsing preparations in the form of an aqueous preparation with a content of 0.1 to 10 % by weight of polyaldehydrocarboxylic acids which contain monomer units corresponding to general formulae I and II

$$-CH_2-\underset{CHO}{\underset{|}{CH}}- \quad (I) \qquad -CH_2-\underset{COOH}{\underset{|}{CH}}- \quad (II)$$

and which have an average molecular weight of 600 to 10,000 and contain from 5 to 9 and preferably from 7 to 9 carboxyl groups and from 1 to 5 and preferably from 1 to 3 aldehyde groups per 10 monomer units or water-soluble salts thereof, characterized in that they additionally contain from 0.1 to 10 % by weight of an anionic hair-conditioning component selected from compounds corresponding to the general formulae $RO(CH_2—CH_2O)_nPO_3A_2$, $[RO(CH_2CH_2O)_n]_2PO_2A$, $R—PO_3A_2$, $R—CH(OH)PO_3A_2$, $RCX(PO_3A_2)_2$ in which R is a linear alkyl group containing from 8 to 18 carbon atoms, X represents hydrogen, an OH-group or an $NH_2$-group, A is an alkali, ammonium, mono-, di- or triethanolammonium cation and n is 0 or an integer of from 1 to 12.

3. Preparations as claimed in Claim 2 for use as shampoos, characterized in that they contain from 1 to 10 % by weight of the polyaldehydocarboxylic acid and from 5 to 50 % by weight of anionic surfactants selected from compounds corresponding to the general formulae $R—SO_3—A$ and/or $RO(CH_2—CH_2—O)_n—SO_3A$, in which R is a linear alkyl group containing from 8 to 18 carbon atoms, A is an alkali, ammonium or alkanolammonium cation and n is 0 or an integer of from 1 to 12.

4. Preparations as claimed in Claim 3, characterized in that they contain a $C_{12}$-$C_{16}$-fatty alcohol (poly) glycol eher sulfate sodium salt containing from 1 to 6 glycol ether groups in a quantity of from 5 to 20 % by weight as the anionic surfactant and, in addition, an ampholytic surfactant, a betaine surfactant or an amine oxide surfactant in a quantity of from 1 to 10 % by weight.

5. Preparations as claimed in Claim 2 for use as hair rinses, characterized in that they contain from 0.1 to 10 % by weight of the polyaldehydocarboxylic acid and from 1 to 10 % by weight of an anionic, hair-conditioning component selected from water-soluble salts of phosphoric acid mono- and diesters of linear $C_{12}$-$C_{18}$-fatty alcohols and/or of $C_8$-$C_{18}$-alkane-1-phosphonic acids.

## Revendications

1. Utilisation d'une préparation aqueuse renfermant des acides polyaldéhydocarboxyliques avec des motifs monomères des formules générales I et II,

$$-CH_2-\underset{CHO}{\underset{|}{CH}}- \quad (I) \qquad -CH_2-\underset{COOH}{\underset{|}{CH}}- \quad (II)$$

possédant un poids moléculaire moyen de 600 à 10 000, et présentant 5 à 9, de préférence 7 à 9, groupements carboxyle, et 1 à 5, de préférence 1 à 3, groupements aldéhyde par 10 unités monomères, ou emploi de leurs sels hydrosolubles pour le lavage ou le rinçage des cheveux.

2. Produit pour le lavage ou le rinçage des cheveux sous la forme d'une préparation aqueuse renfermant 0,1 à 10 % en poids d'acides polyaldéhydocarboxyliques avec des motifs monomères des formules générales I et II,

$$-CH_2-\underset{CHO}{\underset{|}{CH}}- \quad (I) \qquad -CH_2-\underset{COOH}{\underset{|}{CH}}- \quad (II)$$

possédant un poids moléculaire moyen de 600 à 10 000, et présentant 5 à 9, de préférence 7 à 9, groupements carboxyle, et 1 à 5, de préférence 1 à 3, groupements aldéhyde par 10 unités monomères, ou

leurs sels hydrosolubles, caractérisé en ce qu'il renferme en outre 0,1 à 10 % en poids d'un composant anionique avivant la chevelure, choisi parmi des composés des formules générales $RP(CH_2—CH_2O)_n—PO_3A_2$, $[RO(CH_2CH_2O)_n]_2PO_2A$, $R—PO_3A_2$, $R—CH(OH)PO_3A_2$, $RCX(PO_3A_2)_2$, dans lesquelles R représente un groupement alkyle linéaire comportant 8 à 18 atomes de carbone, X de l'hydrogène, un groupement OH ou un groupement $NH_2$, A est un cation de métal alcalin, d'ammonium, de mono-, de di- ou de triéthanolammonium, et n = 0 ou un nombre entier de 1 à 12.

3. Produit selon la revendication 2 pour utilisation comme shampooing, caractérisé en ce qu'il renferme 1 à 10 % en poids d'acide polyaldéhydocarboxylique et 5 à 50 % en poids de tensioactifs anioniques choisis parmi des composés des formules générales $R—SO_3—A$ et/ou $RO(CH_2—CH_2O)_n—SO_3A$, dans lesquelles R représente un groupement alkyle linéaire comportant 8 à 18 atomes de carbone, A est un cation de métal alcalin, d'ammonium, ou d'alcanolammonium et n = 0 ou un nombre entier de 1 à 12.

4. Produit selon la revendication 3, caractérisé en ce qu'il renferme comme tensioactif anionique un alcool gras (en $C_{12}$-$C_{16}$)-polyglycoléther-sulfate-sel sodique, comportant 1 à 6 groupements glycoléther dans une proportion de 5 à 20 % en poids et en outre, un agent de surface ampholyte, un surfactif bétaïne ou un tensioactif à l'oxyde d'amine dans une proportion de 1 à 10 % en poids.

5. Produit selon la revendication 2 pour utilisation comme produit de rinçage final des cheveux, caractérisé en ce qu'il renferme 1 à 10 % en poids d'un composant anionique avivant la chevelure, choisi parmi les sels hydrosolubles des mono- et diesters d'acide phosphorique des alccols gras linéaires de $C_{12}$ à $C_{18}$ et/ou des acides alcane (en $C_8$-$C_{18}$)-1-phosphoniques.